(19) Europäisches Patentamt
European Patent Office
Office européen des brevets

(11) Numéro de publication : **0 549 433 A1**

(12) # DEMANDE DE BREVET EUROPEEN

(21) Numéro de dépôt : **92403466.3**

(51) Int. Cl.[5] : **A61K 31/40,** A61K 31/18

(22) Date de dépôt : **21.12.92**

(30) Priorité : **24.12.91 FR 9116093**

(43) Date de publication de la demande :
**30.06.93 Bulletin 93/26**

(84) Etats contractants désignés :
**AT BE CH DE DK ES FR GB GR IE IT LI LU NL PT SE**

(71) Demandeur : **ADIR ET COMPAGNIE**
**1 rue Carle Hébert**
**F-92415 Courbevoie Cédex (FR)**

(72) Inventeur : **Wierzbicki, Michel**
**8 Résidence du Chemin Pavé**
**F-78620 l'Etang La Ville (FR)**
Inventeur : **Hugon, Pierre**
**13 rue Eugène Sue**
**F-92500 Rueil Malmaison (FR)**
Inventeur : **Duhault, Jacques**
**14bis rue Paul Demange**
**F-78290 Croissy Sur Seine (FR)**
Inventeur : **Espinal, Joseph**
**11 rue de Chateaubriand**
**F-75008 Paris (FR)**

(74) Mandataire : **Reverbori, Marcelle**
**Adir et Compagnie, 1, rue Carle Hébert**
**F-92415 Courbevoie Cédex (FR)**

(54) **Utilisation de N-phenylsulfonyl urées N'-substituées dans le traitement de l'hypertension chez des sujets insulino-resistants.**

(57) Utilisation pour l'obtention de médicaments destinés au traitement de l'hypertension chez des sujets insulino-résistants,
— de dérivés de formule :

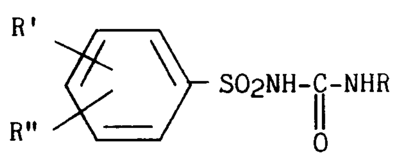

dans laquelle R, R' et R" sont tels que définis dans la description,
— et de leurs sels physiologiquement tolérables.

EP 0 549 433 A1

La présente invention concerne l'utilisation de N-phénylsulfonylurées N'-substituées pour l'obtention de médicaments destinés au traitement de l'hypertension chez des sujets présentant une insulino-résistance associée ou non à d'autres anomalies métaboliques.

Plus spécifiquement, l'invention concerne l'utilisation, pour l'obtention de médicaments destinés au traitement de l'hypertension chez des sujets présentant une insulino-résistance associée ou non à d'autres anomalies métaboliques, les dérivés de formule générale I :

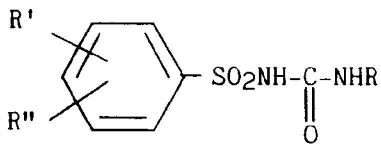

(I)

dans laquelle :

- R représente :
  a) un radical azabicycloalkyle de formule :

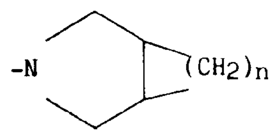

  dans laquelle n est un nombre entier de 1 à 3,
  b) un radical isoindolin-2-yle de formule :

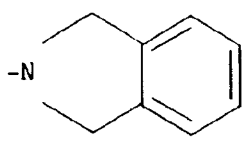

  ou
  c) un radical 1,7,7-triméthyl 2-oxo bicyclo[2,2,1] hept-3 yle de formule :

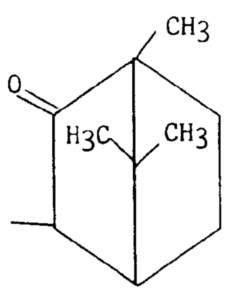

  et
- R' et R", identiques ou différents, représentent chacun :
  - un atome d'hydrogène,
  - un atome d'halogène,
  - un radical alkyle ou alkoxy de 1 à 5 atomes de carbone en chaine droite ou ramifiée, ou
  - un radical nitro,

et leurs sels physiologiquement tolérables.

Les dérivés de formule générale I et leurs sels ont notamment fait l'objet des brevets français n° 1.510.714, 1.555.074 et du certificat d'addition français n° 95.283.

La demanderesse a présentement découvert que les dérivés de formule I et leurs sels physiologiquement

tolérables, bien que dépourvus d'activité hypotensive intrinsèque, diminuent la pression artérielle des sujets insulino-résistants et de ce fait peuvent être utilisés en thérapeutique dans le traitement de l'hypertension associée aux états d'insulino-résistance et autres anomalies métaboliques telles que par exemple obésité, dyslipémie, hyperinsulinémie etc... qui constituent des facteurs de risques cardiovasculaires importants (coronaropathies, macroangiopathie, etc...).

La présente invention a également pour objet les compositions pharmaceutiques contenant comme principe actif un dérivé de formule I ou un de ses sels physiologiquement tolérables, mélangé ou associé à un excipient pharmaceutique approprié.

Les compositions pharmaceutiques ainsi obtenues sont présentées avantageusement sous des formes convenant pour l'administration par voie orale, ou parentérale telles que par exemple comprimés, dragées, gelules, solutions injectables ou buvables.

Les formes dosées renferment de 15 à 150 mg de principe actif.

La posologie varie selon l'âge et le poids du patient, la voie d'administration, la nature des traitements associés et s'échelonne de 15 à 150 mg de principe actif par prise, 1 à 3 fois par jour.

L'étude pharmacologique suivante illustre la présente invention sans toutefois la limiter.

## ETUDE PHARMACOLOGIQUE

### Etude du traitement chronique du rat âgé

Au cours du vieillissement, une insulino-résistance apparaît, accompagnée d'une hyperinsulinémie et d'une élévation de la pression artérielle (cf tableau 1 ci-après).

Tableau 1

| | Insulinémie µU/ml | Pression artérielle $10^2$ Pa |
|---|---|---|
| **Rats 3 mois** | 16 ± 1 | 133 ± 9 |
| **Rats 12 mois** | 32 ± 3 | 170 ± 8 |

A titre d'exemple, le traitement par le gliclazide à la dose de 0,1 à 1 mg /kg par jour pendant 2 semaines, diminue la pression artérielle du rat âgé de $9.10^2$ à $11.10^2$ Pa sans modifier celle du rat jeune.

L' étude ci-dessus montre l'intérêt de l'utilisation des dérivés I et tout principalement du gliclazide pour l'obtention de médicaments destinés au traitement de l'hypertension chez des sujets présentant une insulino-résistance associée ou non à d'autres anomalies métaboliques.

## Revendications

**1-** Utilisation, pour l'obtention de médicaments destinés au traitement de l'hypertension chez des sujets insulino-résistants, des dérivés de formule générale I :

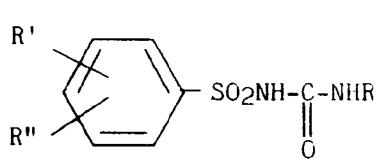

(I)

dans laquelle :

- R représente :
  a) un radical azabicycloalkyle de formule :

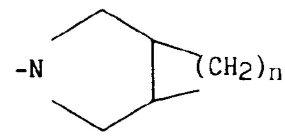

  dans laquelle n est un nombre entier de 1 à 3,
  b) un radical isoindolin-2-yle de formule :

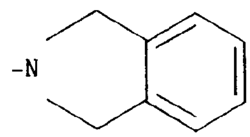

  ou
  c) un radical 1,7,7-triméthyl 2-oxo bicyclo[2,2,1] hept-3 yle de formule :

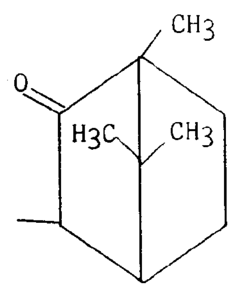

et
- R' et R", identiques ou différents, représentent chacun :
  - un atome d'hydrogène,
  - un atome d'halogène,
  - un radical alkyle ou alkoxy de 1 à 5 atomes de carbone en chaine droite ou ramifiée, ou
  - un radical nitro,

ainsi que leurs sels physiologiquement tolérables.

**2-** Utilisation, selon la revendication 1, pour l'obtention de médicaments destinés au traitement de l'hypertension chez des sujets insulino-résistants, du gliclazide.

**3-** Les compositions pharmaceutiques pour le traitement de l'hypertension chez des sujets insulino-résistants, renfermant comme principe actif au moins un dérivé de formule I définie dans la revendication 1 ou un de ses sels physiologiquement tolérables.

**4-** Les compositions pharmaceutiques pour le traitement de l'hypertension chez des sujets insulino-résistants, renfermant comme principe actif le gliclazide.

**5-** Les compositions pharmaceutiques, selon chacunes des revendications 3 et 4, caractérisées en ce qu'elles renferment de 15 à 150 mg de principe actif, mélangé ou associé à un excipient pharmaceutique approprié.

Office européen des brevets

# RAPPORT DE RECHERCHE EUROPEENNE

Numero de la demande
EP 92 40 3466

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| Y | JPN.PHARMACOL.THER.<br>vol. 17, no. 1, 1989,<br>pages 313 - 319<br>* abrégé *<br>* page 316; tableau 1 * | 1-2 | A61K31/40<br>A61K31/18 |
| Y | AM.J.MED.<br>vol. 90, no. 6A, Juin 1991,<br>pages P3S - P7S<br>* page 3S, colonne de droite, ligne 48 - page 4S, colonne de gauche, ligne 11 *<br>* page 6S, colonne de gauche, ligne 29 - ligne 34 * | 1-2 | |
| Y | DIABETES CARE<br>vol. 13, no. SPL3, 1990,<br>pages 53 - 58<br>* page 54 *<br>* page 55, colonne de gauche, ligne 26 - ligne 28 * | 1-2 | **DOMAINES TECHNIQUES RECHERCHES (Int. Cl.5 )** |
| Y | POSTGRAD.MED<br>vol. 89, no. 4, Mars 1991,<br>pages 65 - 72<br>* abrégé *<br>* page 67, colonne 1, ligne 1 - ligne 6 *<br>* page 71, colonne 2, ligne 16 - ligne 28 * | 1-2 | A61K |
| X | 'The Merck Index'<br>1987 , MERCK & CO.<br>Gliclazide<br>* page 696 * | 3-5 | |

Le présent rapport a été établi pour toutes les revendications

| Lieu de la recherche | Date d'achèvement de la recherche | Examinateur |
|---|---|---|
| LA HAYE | 11 MARS 1993 | GERLI P.F.M. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)